Europäisches Patentamt

European Patent Office

Office européen des brevets

(11) Publication number: **0 439 354 A2**

(12)

# EUROPEAN PATENT APPLICATION

(21) Application number: **91300542.7**

(22) Date of filing: **24.01.91**

(51) Int. Cl.⁵: **C12Q 1/70, C12Q 1/68,**
**G01N 33/569, // G01N33/68,**
**G01N33/58, C12Q1/66 ,**
**(C12Q1/68, C12R1:19)**

(30) Priority: **24.01.90 US 469334**

(43) Date of publication of application :
**31.07.91 Bulletin 91/31**

(84) Designated Contracting States :
**AT BE CH DE DK ES FR GB GR IT LI LU NL SE**

(71) Applicant : **AMOCO CORPORATION**
**200 East Randolph Drive**
**Chicago Illinois 60601 (US)**

(72) Inventor : **Bittner, Michael Lewis**
**1768 Brookdale Road**
**Naperville, Illinois 60563 (US)**
Inventor : **Müller, Uwe Richard**
**11714 River Road**
**Plano, Illinois 60545 (US)**
Inventor : **Beman, John David**
**232 Bastian Drive**
**Sugar Grove, Illinois 60554 (US)**
Inventor : **Taron, Douglas James**
**469 River Bluff Road**
**Elgin, Illinois 60120 (US)**

(74) Representative : **Laredo, Jack Joseph et al**
**Elkington and Fife Beacon House 113**
**Kingsway**
**London, WC2B 6PP (GB)**

(54) **Signal generating moiety and method for use.**

(57)    A self-amplifying signal generating moiety and an assay method for detecting minute amounts of target molecules are disclosed. The signal generating moiety includes an active bacteriophage or virus and detector probe. After contacting with the target, the signal generating moiety is introduced to a suitable host and then allowed to replicate and produce an amplified signal.

EP 0 439 354 A2

## SIGNAL GENERATING MOIETY AND METHOD FOR USE

### Field of the Invention

The present invention relates to a self-amplifying signal generating moiety for use in an assay. More particularly, the present invention relates to a signal generating moiety for use in any assay wherein the concentration of the assay target is extremely low and an amplified signal is produced in the assay which is useful in detecting the presence and/or concentration of the target. The present invention provides an assay that enables the detection and/or quantification of molecules present in $10^{-18}$ mole amounts or less.

### Background of the Invention

Numerous assays employing various means for producing a detectable signal are known to the art. The assays known to date, however, produce signals of limited strength and, hence, are of limited sensitivity. The more sensitive of the known assays combine a signal generating moiety (a label) with a detector probe to form a complex which binds with the target of the assay. The signal generating moieties are typically fluorescent moieties or enzymes. The detector probes are typically antibodies, avidins or sequences of nucleic acid.

Assays for specifc DNA or RNA sequences (DNA probe assays) permit especial selectivity relative to assays for other targets. DNA probe assays are particularly useful in detecting pathogens and other microbiological agents and DNA or RNA sequences resulting from genetic diseases. Current DNA probe applications are, however, limited by the sensitivity of the assay technique. DNA probe assays using fluorescing labels are typically sensitive to about $10^{-17}$ moles (per assay volume) of target molecule. Assays using enzymatic labels have been developed which are sensitive to about $10^{-18}$ moles (per assay volume).

Bacteriophages are able to replicate when introduced to a suitable host cell and have been used in a competitive binding assay by Young in U.S. No. 4,104,126. Young further discloses the use of certain intracellular constituents, such as enzymes, to determine the quantity of target present. However, the assay is less sensitive than the $10^{-18}$ mole sensitivity of assays using the most sensitive enzymatic labels. Moreover, the bacteriophages used in that assay are purposefully inactivated by binding proteins specific to the substrate being assayed.

U.S. Patent No. 4,746,604 to Moskowitz further discloses the use of a viable cell as the signal generating moiety in several assay formats. The cells are permitted to replicate and thereby produce an amplified signal. U.S. Patent No. 4,746,604 does not disclose the use of bacteriophages or viruses in any of the assay formats and does not disclose assay sensitivity to $10^{-18}$ moles or less.

Accordingly, it is an object of the present invention to provide a noncompetitive binding assay which permits the detection or quantification of molecules present in $10^{-18}$ mole amounts or less. It is another object of the present invention to provide an assay for detection of minute amounts of target molecules using a self-amplifying signal generating moiety in the assay. It is still a further object of the invention to use an active virus or bacteriophage in a self-amplifying signal generating moiety in an assay for detecting minute amounts of DNA or RNA sequences. The phage or virus itself can be used to determine the amount of assay target present, or can be used to produce an enzyme or other catalyst which is then used to determine the amount of target present. These and other objects of the invention and the manner in which they are obtained will be clear from the detailed description of the invention disclosed herein.

### Summary of the Invention

The present invention has three aspects. The first aspect is a self-amplifying signal generating moiety which can be used in an assay to detect the presence or concentration of minute amounts of a target molecule. In particular, the signal generating moiety comprises a vector which serves as a source of nucleic acid and a detector probe. The vector is typically a bacteriophage or virus and must be suitable for receipt by a host and reproduction therein. The host is typically a cell that has been selected for compatibility with the vector. The nucleic acids produced by the vector can also contain one or more sequences of nucleotides encoding for a polypeptide which can be detected directly or can participate in a subsequent reaction to produce a detectable signal. The polypeptide can be a protein, enzyme, cofactor or the like. The nucleotide sequence is translated by the host to produce the polypeptide. Hence, the vector produces an amplified signal. The detector probe of the signal generating moiety binds the vector with the target to form a reporter complex comprising the vector, the detector probe and the target.

The second aspect of the present invention is a method for detecting the presence and/or the concentration of a target molecule using the signal generating moiety of this invention. The reporter complex can be isolated from uncomplexed vector and detector probes by conventional procedures. The isolated reporter complex or, more particularly, the vector component thereof can then be introduced to a host where the vector can reproduce and produce a detectable signal. The nucleic acids produced by the vector may also encode for a polypeptide. In this case, the vector is introduced to the host to reproduce and further produce the polypeptide for the signal generating reaction. More generally, the method comprises the steps of :

(a) contacting the signal generating moiety with the target to form a reporter complex ;

(b) separating the reporter complex from unbound components of the signal generating moiety ;

(c) introducing the vector from the separated reporter complex to a suitable host ;

(d) allowing the vector to produce a signal ; and

(e) detecting the signal.

Once the reporter complex has been separated from unbound components, it can be introduced to the host. Alternatively, the reporter complex can be separated into various components. It is only necessary that the vector be introduced to the host to produce the amplified signal. It is an advantage of the invention that when a phage or virus is used as the vector, the phage or virus can insert itself or its nucleic acids into the host.

The method can be used merely to detect the presence or absence of the target molecule. It can also be used to quantitate the concentration of the target present. It is an advantage of the signal generating moiety that, when introduced to the host, the signal generating moiety produces an amplified signal which can be correlated to the amount of target present. When used in the method of the invention, the method provides greatly enhanced sensitivity over conventional detection methods.

The third aspect of the present invention is to incorporate the signal generating moiety and the method into a test kit for the detection of a specified target. The test kit would comprise a signal generating moiety or its components and means for accessing the system to be tested. The kit would also comprise means for separating the nucleic acid source, which has bound to the target, from unbound components of the signal generating moiety. The kit would further provide means for introducing the isolated nucleic acid source into a suitable host and may also include means for detecting the signal that results therefrom.

## Detailed Description of the Invention

The present invention is directed to a self-amplifying signal generating moiety and a method for detecting the presence and/or concentration of a target molecule. The signal generating moiety and detection method can be used to detect molecules, ions and complexes. The invention is used most advantageously in the detection of biomolecules such as proteins, polypeptides, nucleic acids, hormones, haptens, antigens, antibodies and the like which are used or generated in a biological process. More particularly, the invention can be used to detect RNA or DNA sequences of microbiological agents or sequences which result from genetic diseases.

The self-amplifying signal generating moiety enables detection of the target by binding to the target and producing a material able to produce a signal or by producing the signal itself. The intensity of the resulting signal is directly related to the concentration of the target. Hence, standardization of the signal intensity enables determination of the concentration of the target. The extent of signal amplification afforded by the invention is such as to permit detection and quantification of targets at concentrations that were heretofore impossible to detect. For example, the invention has permitted detection of fewer than $10^4$ target molecules. Accordingly, less than $10^{-18}$ mole amounts per assay volume of target are now detectable. While the invention can be used in any format, it is more advantageous to use it in an assay wherein the amount of the target present is about $6 \times 10^{-18}$ moles (per assay volume) or less.

The signal generating moiety comprises a vector and a detector probe. The vector serves as a source of nucleic acids and can be naturally occurring or genetically modified bacteriophages, viruses, plasmids or genetic modifications thereof. The nucleic acids produced by the vector are DNA, RNA, other polypeptides or fragments thereof, and can be naturally occurring or synthetic sequences.

The vector must, however, be capable of introduction to and replication by a host. The vector and the host are, therefore, normally selected for compatibility. Bacteriophages and viruses are preferred vectors because they are able to infect cells, thereby introducing themselves or their nucleic acids into the cells. Typically, bacterial cells such as $\underline{E}$. $\underline{coli}$ are used when the vector is a bacteriophage and mammalian cells are used when the vector is a retrovirus.

Additionally, the nucleic acid may contain one or more nucleotide sequences which encode for a polypeptide that provides the signal to be detected. The polypeptide can be any substance which can produce the signal itself or participate in a reaction that produces the signal. Proteins, enzymes, cofactors and the like are typical polypeptides for use in the present invention. Normally, the nucleic acid is genetically modified to include a nucleotide sequence for generating a suitable polypeptide.

3

Enzymes which can catalyze the formation of a measurable product are preferred. Suitable enzymes include the acid phosphatases, the alkaline phosphatases, the peroxidases, the galactosidases, the luciferases and the like. These enzymes can catalyze the production of light (photons), chromophores, precipitates and the like. These enzymes can also catalyze the removal of a measurable substance from the reaction system. For example, they are able to convert chromophores into nonchromophores. These enzymes are also able to convert insoluble particulates into soluble substances thereby permitting removal of the insoluble particulates from a suspension.

Luciferase enzymes catalyze the emission of light and so are preferred. Typical luciferases include that from the bacterium Vibrio fischeri and that from the firefly Photinus pyralis. The luciferase from the bacterium Vibrio harveyi is especially preferred.

The self-amplifying signal generating moiety of the present invention also comprises a detector probe. The detector probe binds to both the target and the vector thereby linking the two together. This linkage permits detection and/or quantification of the target molecule. The detector probe thus comprises one or more components which together are able to bind to both the vector and the target. The binding is sufficient if the three join to form a vector-detector probe-target complex (a reporter complex) which can be separated from its unbound components. The detector probe can be selected so as to bind to the target and vector by any form of binding including covalent bonds, ionic bonds, hydrogen bonds, hybridization, electrostatic attractions, van der Waals' forces and the like. The detector probe can bind to the target by the same or a different mechanism as it binds to the vector.

Typically, one component of the detector probe is a polynucleotide chain which contains a sequence of nucleotides able to hybridize with the target. While the polynucleotide chain may also contain a sequence able to hybridize with the vector, more typically binding to the vector is facilitated through the use of binding pairs such as mono- and polyclonal antibodies, fragments thereof and their corresponding antigens and haptens. These include antigen-Fab, antigen-Fab', antigen-F(ab')$_2$, hapten-Fab, hapten-Fab', hapten-F(ab')$_2$, antigen-antibody and hapten-antibody pairs. Other suitable binding pairs include biotin or biotinylated DNA polynucleotides with anti-biotin, DNP on dinitrophenylated proteins with anti-DNP, lectin-glycoprotein, biotin-avidin, biotin-streptavidin, complementary nucleic acid sequences and the like. The detector probe may comprise more than one binding pair.

One member of the binding pair can be bound directly to the vector while the other member of the pair is bound to another element of the detector probe (e.g., a polynucleotide chain). For example, when the vector is a bacteriophage or a virus, it is advantageous to bind one member of a binding pair to the phage before binding with the other member of the pair in the detector probe. Binding to a phage or virus can be facilitated by modifying the surface of the phage or virus to introduce functional groups able to bind to a binding pair member. Free carboxyl groups are advantageous for such binding ; free amino groups are especially useful. Examples 1 and 2 herein illustrate the use of a modified M13 bacteriophage as a vector. In both examples, biotin was bound to the surface of the phage to facilitate binding with a polynucleotide chain via its binding partner, streptavidin. The biotin, streptavidin and polynucleotide chain are all considered components of the detector probe. Similarly, in Example 4, the F(ab') monomer of anti-fluorescein antibody was bound to the surface of a modified M13 bacteriophage for binding with fluorescein of the detector probe. Both fluorescein and the F(ab') anti-fluorescein monomer are considered components of the detector probe.

Various binding mechanisms are possible when one or both members of a binding pair are multivalent. For example, in Examples 1 and 2 biotin was bound separately to a polynucleotide chain and to the bacteriophage. Streptavidin, which is a multivalent binding partner to biotin, completed the detector probe by binding to the biotin on both the phage and the polynucleotide chain. Other binding mechanisms will be readily apparent to those skilled in this art.

The self-amplifying signal generating moiety permits enhanced amplification of a detectable signal at several levels. The first level results when the vector is introduced to the host and replicates thereafter. For example, after introduction to a host cell bacteriophages such as M13 and $\phi$X174 typically replicate their genome to produce hundreds of copies within the host cell. Further, many of the genomes are released into the culture medium surrounding the host cell and go on to infect still other host cells. Each newly infected host repeats the process to produce hundreds more of additional copies of the original phage genome. The increase in the number of phage over time is exponential. For example, the modified M13 bacteriophage employed in Examples 1, 2 and 4 increased in number by a factor greater than $10^4$ in 2 hours in standard E.coli cells under the conditions used therein. A second level of amplification results when the nucleic acids produced by the vector encode for a polypeptide. This amplification results from transcription of the nucleic acids to produce hundreds to thousands of mRNA transcripts. These are then multiply-translated to produce even more polypeptides. Still greater amplification is possible when the product polypeptide participates in a reaction that produces a signal which accumulates in time.

In another aspect of the invention, the self-amplifying signal generating moiety is used in a method for determining the presence or concentration of a target. The method comprises the steps of :

(a) contacting the signal generating moiety with the target to form a signal generating moiety-target complex ;

(b) separating the signal generating moiety-target complex from uncomplexed components of the signal generating moiety ;

(c) introducing the vector from the separated signal generating moiety-target complex into a suitable host;

(d) allowing the vector to produce an amplified signal ; and

(e) detecting the signal.

The resulting signal can be detected vis-a-vis a specifed threshold to determine the presence or absence of the target. Alternatively, the signal can be measured vis-a-vis the signals of known standards to determine the concentration of the target.

The signal generating moiety is contacted with and allowed to bind with the target. It is not intended, however, to require that the signal generating moiety be completely formed before being contacted with the target. The signal generating moiety-target complex can be formed in any manner which results in a completed complex. In Examples 1 and 2, for example, the detector probe comprised a polynucleotide chain, streptavidin and two molecules of biotin. In each example, the polynucleotide chain of the detector probe was first biotinylated, then contacted with the target, then bound to streptavidin and only then bound to the vector, which itself had first been biotinylated. Those skilled in the art will readily appreciate alternative protocols for forming the signal generating moiety-target complex.

The signal generating moiety and target are typically contacted in an aqueous phase. By aqueous phase is meant a solvent phase that is from about 90% to about 100% water with the remainder comprising one or more water miscible organic solvents that do not denature or otherwise adversely affect the components or the binding among the components of the signal generating moiety and the target. Suitable solvents include, but are not limited to, low molecular weight alcohols having from 1 to 4 carbon atoms, and polyols (e.g., polyethylene glycols) and polyethers (e.g., polyvinyl pyrrolidones) with molecular weights of from about 100 to about 10,000.

Typically, target molecules bind to the detector probe via hybridization of complementary nucleotide sequences in one component of the detector probe and the target molecule. The signal generating moiety-target complex is then separated from the uncomplexed materials (e.g., uncomplexed signal generating moiety or components parts). The separation can be accomplished by conventional means including, but not limited to, precipitating the complex from the aqueous phase, binding it to a solid phase support which is then removed from the aqueous phase, or any other suitable means. Preferably, the complex is bound to a solid phase support (solid phase).

By solid phase is meant any solid phase support not soluble in the aqueous phase. Conventional solid phase materials include metals, glasses and plastics. The solid phase can be the vessel wherein the contacting takes place (e.g., a test tube, a centrifugation cup, a microtiter cup or the like) or a device inserted into the vessel (e.g., beads or a dipstick). Metal and plastic are preferred materials for beads ; glass and plastic are preferred for vessels.

More particularly, any plastic to which a binding pair member may attach is suitable for use as a solid phase. The plastics may be hydrophobic (e.g., polystyrene, polyethylene and polypropylene) or hydrophilic (e.g., plastics comprising sulfate, sulfonate, carboxylic acid, primary amine, aromatic amine, amide, hydroxyl, tosyl activated or chloromethyl groups). Polyethylene, polystyrene, polypropylene, and copolymers of styrene/vinyl carboxylic acid, styrene/aromatic amine and styrene/primary amine are preferred. Such materials are available in microspheres or particulates from Bang's Laboratories, Carmel, Indiana.

Binding of the signal generating moiety-target complex to a solid phase is often facilitated through a capture probe, which functions in the same manner as the detector probe. The capture probe comprises one or more components which can bind to both the target and the solid phase. The binding is sufficient if it permits the signal generating moiety-target complex to be separated from uncomplexed components. The capture probe can bind to the target by covalent bonds, ionic bonds, hydrogen bonds, hybridization, electrostatic attractions, van der Waals' forces and the like.

Typically, one component of the capture probe is a polynucleotide chain which binds to the target. Binding occurs through hybridization of complementary nucleotide sequences in the target and the polynucleotide chain. The chain is selected, however, to bind to a sequence in the separate target from that bound by the detector probe to avoid interfering with the binding by that probe.

The capture probe can be bound to the solid phase directly. However, such binding can also be facilitated by using binding pairs similar to those used to facilitate binding of the vector to the detector probe. These include monoclonal and polyclonal antibodies and fragments thereof and their corresponding antigens and haptens. Suitable binding pairs include antigen-Fab, antigen-Fab', antigen-F(ab')$_2$, antigen-antibody , hapten-Fab, hapten-Fab', hapten-F(ab')$_2$ and hapten-antibody in general, and biotin or biotinylated DNA probes with anti-biotin, DNP on dinitrophenylated proteins with anti-DNP, lectin-glycoprotein, biotin-avidin, biotin-streptavidin, complementary nucleic acid sequences and the like in particular. The capture probe may include more than one binding pair.

As was the case with the signal generating moiety, it is not intended that the binding between the target, capture probe and solid phase occur in any particular order. In Examples 1 and 2, for example, a complex between the polynucleotide chain of the detector probe, the target and a polynucleotide chain of the capture probe was allowed to form before the complex was immobilized by introducing a solid phase. A second polynucleotide chain of the capture probe had previously been bound to the solid phase. Thereafter, a binding pair member was added to complete the detector probe which was then followed by introducing the vector to complete the protocol. Those skilled in the art will readily appreciate alternative protocols for binding the signal generating moiety to a solid phase.

The immobilized signal generating moiety-target complex is then separated from any remaining unbound components. Separation can be accomplished by decanting, aspirating, blotting, wicking away the aqueous phase or the like, or by simply removing the solid phase (e.g., beads or dipstick). The separation can be enhanced by washing with appropriate wash solutions. The signal generating moiety-target complex is then introduced to and allowed to infect the host cell using techniques that are well known to those skilled in the art. Alternatively, the vector can be separated from the bound complex and introduced into the host.

Thereafter, the vector will produce an amplified signal as previously described. When only the presence of the target is desired, the signal is measured to determine whether a threshold level is produced. If the threshold is obtained, the response is positive ; if not, the target is deemed not present. Comparison of the measured response against known standards permits determination of the concentration of target.

The invention and reagents used for conducting the method of the invention can be incorporated into kits. The kits would comprise, in part, components of the self-amplifying signal generating moiety, means for accessing the system to be tested, and means for separating the vector of the signal generating moiety from unbound components once the signal generating moiety has been allowed to bind to the target. The kit would further provide means for introducing the isolated vector to a suitable host and may also include means for detecting the signal that results therefrom.

Examples

The following examples illustrate various embodiments of the present invention. Each example illustrates an assay with a ribosomal RNA as the target. The sensitivity of each assay is expressed in cell equivalents and moles of target per assay. The bacteria used in the examples typically contain about $5 \times 10^4$ ribosomes per cell and this number was used to determine the molar sensitivity of each assay for target rRNA. The examples are not intended to limit the scope of the invention in any way. The amounts of bacteriophage used in the examples are expressed in plaque-forming units (pfu) and were determined using the well known plaque assay.

Example 1

This example illustrates a signal generating moiety and its use in detecting the pathogen Listeria innocua. In the example, the signal generating moiety is incorporated into a dual probe assay (i.e., an assay using detector and capture probes) using magnetic beads as the solid phase. E.coli was used as the host.

A modified M13 bacteriophage was used as the vector. In particular, the luxA and luxB genes of the luciferase enzyme from the bacterium Vibrio harveyi were introduced into the bacteriophage M13mp19. The nucleotide sequences of luxA and luxB are taught in Cohn et al., J. Biological Chem., 260 :6139-6146 (1985) and Johnston et al., J. Biological Chem., 261 :4805-4811 (1986), respectively. The genes were obtained as a 1929 base pair Sal-I to Bg1-II restriction fragment from plasmid pTB7. The plasmid is available in strain NRRLB-15231 from the USDA NRRL in Peoria, IL. The restriction fragment was inserted into the bacteriophage M13mp19 between the Sal-I and BamH-I restriction sites. M13mp19 is available from Bethesda Research Labs, Gaithersburg, MD. All cloning manipulations were carried out by standard methods and can be readily reproduced by those with skill in the art. The resulting bacteriophage M13SOC512 contained the coding sequences for luxA and luxB under the transcriptional control of the lac promoter present in M13mp19.

Biotin was then bound to the surface of the M13SOC512 phage by suspending the phage in phosphate buffer (0.1 M NaH$_2$PO$_4$, 0.1 M NaCl, pH 7.5) with N-hydroxysuccinimide biotin (NHS-LC-biotin) for 60 min at 25°C. The optimized reaction mixture contained from about $1 \times 10^{13}$ to about $5 \times 10^{13}$ pfu of M13SOC512 and about 210 µg NHS-biotin in a volume of about 5 ml (pH 7.5). Plating assays were used to determine the number of viable bacteriophage before and after biotinylation. The assays indicated that phage viability was unchanged

6

after biotinylation. The biotinylated phage was separated from unreacted NHS-biotin by Sephadex G-25 chromatography and further concentrated by precipitation in 2% polyethyleneglycol (0.5 M NaCl) and resuspension in 1 ml phosphate buffer. More than 99.9% of the bacteriophage reacted with the NHS-biotin.

The detector probe comprised a synthetic RNA polynucleotide chain, biotin and streptavidin. The RNA chain contained a 575 nucleotide sequence complementary to bases 80 to 648 of the 16S rRNA from E. coli. Biotin was bound to the RNA chain using conventional techniques. Streptavidin bound to the biotin of the detector probe and of the M13SOC512 bacteriophage to complete the detector probe.

The capture probe comprised oligonucleotides of DNA and dT. The DNA oligonucleotide contained a 35 base synthetic oligonucleotide exactly complementary to a region of the Listeria monocytogenes 16S rRNA. The nucleotide sequence is TGT CCC CGA AGG GAA AGC TCT GTC TCC AGA GTG GT. A dA tail of approximately 125 residues was added at the 3' end by the terminal deoxyribonucleotidyl transferase. The 35 base complementary region does not overlap the region of complementarity for the biotinylated detector probe. The oligo(dT) was also bound to magnetic beads which served as the solid phase of the assay.

The oligo(dT) was attached to the magnetic beads by reacting a 5' amine-terminated oligo(dT) nucleotide with carboxylic acid terminated particles in the presence of 1-ethyl-3(3-dimethylamino-propyl)carbodiimide (EDAC), a water soluble carbodiimide activator. EDAC is available from Sigma Chemical Co. The particles have a mean diameter ranging from 0.7 to 1.7 $\mu$ and are available from Advanced Magnetics, Inc., Cambridge, MA. The oligo(dT) particles are available as part of kits from Gene-Trak Systems, Framingham, MA. The beads were rinsed several times with 0.1 M sodium bicarbonate and several times with sterile water. The beads can be stored in 10 mM EDTA (pH 7.0), 0.05% and 0.02% sodium azide at 4°C until use.

Prior to use, the beads were pretreated twice as follows to reduce nonspecific absorption of cellular materials and labeled probes. The beads were diluted to 0.1% solids with bead prehybridization buffer (0.1 M Tris-HCl (pH 7.5), 10 mM EDTA (pH 7.0), 4% (w/v) bovine serum albumin (BSA, Factor V., Sigma Chemical Co., St. Louis, MO), 0.5% Tween 20) and incubated at 68°C for 3-4 hours in a Pyrex bottle with occasional vigorous agitation. Tween 20 is the trade name for polyoxyethylene sorbitan monolaurate from Sigma Chemical Co. The beads were allowed to cool overnight with little or no agitation. They were then rinsed three times with sterile water and resuspended at 0.1% solids in bead prehybridization buffer plus 0.02% sodium azide at room temperature. The beads were then rotated on a tube rotator at room temperature until use. (Beads have been stored in prehybridization buffer and azide for up to two months before use with no loss in $dA_{12}$ binding capacity.) On the day of use, the beads were washed once with prehybridization buffer and resuspended in the buffer at 0.016% solids for target capture.

The sensitivity of the bead assay for Listeria innocua was evaluated as follows. Cell extracts of Listeria innocua were serially diluted to concentrations of $10^7$, $10^6$, $10^5$ and $10^4$ cells/ml in 0.1 ml of 2.5 M guanidine isothiocyanate (GuSCN) buffer (0.1 M Tris-HCl (pH 7.5), 0.4 M EDTA). To 20 $\mu$l from each dilution was added 12.5 $\mu$l of $10^{-8}$ M DNA oligonucleotide (of the capture probe) and 12.5 $\mu$l of $2\times10^{-9}$ M of biotinylated RNA (of the detector probe). Each solution was brought to pH 7 and incubated for 15 min at 37°C to allow formation of a DNA-rRNA-RNA complex. The rRNA was the 16S rRNA from the cell extracts.

The complexes from each dilution were immobilized by adding 200 $\mu$l of the oligo(dT) magnetic beads (0.1 % solids) and incubating the system for 5 min at 37°C. The beads were then removed from the solution, washed twice in wash buffer (0.5 M NaCl, 0.1 M Tris, 0.5% BSA, 0.1% Tween 20, pH 7.5). The beads were resuspended in 110 $\mu$l wash buffer containing 0.1 $\mu$g streptavidin (Cal-Biochem, San Diego, CA) and incubated for 10 min at room temperature. The beads were then washed twice more in wash buffer and resuspended in 110 $\mu$l wash buffer which contained $10^{10}$ pfu biotinylated M13SOC512 bacteriophage. The suspension was incubated for 10 min at room temperature. The beads were then washed five times in wash buffer and resuspended in 100 $\mu$l thermal elution buffer (0.5 M NaCl, 0.1 M Tris, pH 8.0) which was preheated to 60°C. The beads were incubated for 1 minute at 60°C and then cooled to room temperature by immersing the system in a water bath. The beads were removed from the solution by a magnetic field. Fifty microliter of each supernatant was then assayed for bacteriophage as follows.

The test solutions were added to 1.5 ml Eppendorf tubes. To this was added 200 $\mu$l of mid-log JM101 cells which served as the host and were prepared as follows. A culture of E. coli JM101 (F+) cells was grown overnight at 37°C with aeration. The culture was diluted 1:100 in Luria broth and grown to mid-log phase in about 2 hours. DNAse I was added to a final concentration of $10^3$ Kunitz per ml and RNAse A was added to a final concentration of 1 $\mu$g per ml of culture.

Each combination of test solution and host cells was incubated for 15 min at room temperature without shaking. The contents of each were then added to 2.0 ml of Luria broth containing isopropyl-$\beta$-galactoside at a final concentration of 0.5 mM and incubated at 37°C with aeration for 2 hours.

Thereafter, 200 $\mu$l of each culture was removed and placed in a 12 x 75 mm borosilicate culture tube. Luminescence was induced by injecting 300 $\mu$l of 0.1% solution of decanal in Luria broth. The luminescence was detected using a luminometer set for a 5 second measurement after a 10 second delay, following injection of the decanal. (The luciferase enzyme catalyzes the oxidation of the added decanal with flavin nucleotide endogenous to the system to produce light. The luminometer is from Berthold Analytical Instruments, Inc., Nashua, NH.) System backgrounds were determined by assaying a JM101 culture which was not exposed to bacteriophage. Light output was measured as counts per 5 seconds and plotted against the number of Listeria innocua cells mixed into each dilution.

| Listeria Cell Equivalents | Light Output (light counts/sec) | Signal:Noise |
|---|---|---|
| $5 \times 10^5$ | $3.39 \times 10^6$ | 19.2:1 |
| $5 \times 10^4$ | $1.59 \times 10^6$ | 9.0:1 |
| $5 \times 10^3$ | $1.66 \times 10^5$ | – |
| $5 \times 10^2$ | $1.22 \times 10^5$ | – |
| 0 | $1.77 \times 10^5$ | – |

The assay was sensitive to about $5 \times 10^4$ cell equivalents of Listeria innocua or about $4 \times 10^{-15}$ moles of rRNA.

Example 2

Example 2 illustrates the invention in a dipstick assay. As in Example 1, the target was the 16S rRNA from Listeria innocua. Also used, as in Example 1, were the same biotinylated RNA and streptavidin for the detector probe and the same DNA and oligo(dT) nucleotides for the capture probe. The vector was the same modified M13SOC512 bacteriophage as used in Example 1. Dipsticks were used as the solid phase instead of magnetic beads. The dipstick were coated with the oligo(dT) by Gene-Trak Systems and are available commercially in test kits from Gene-Trak.

The sensitivity of the dipstick assay was determined as follows. Cell extracts of Listeria innocua were serially diluted to concentrations of $10^8$, $10^7$, $10^6$, $10^5$, $10^4$, $10^3$ and $10^2$ cells/ml in 0.1 ml of 2.5 M GuSCN buffer. To 0.3 ml of 2.5 M GuSCN buffer was added 70 µl from each dilution, 12.5 µl of $10^{-8}$ M DNA oligonucleotide and 12.5 µl of $2 \times 10^{-9}$ M biotinylated RNA. Each solution was brought to pH 7 and incubated for 15 min at 37°C.

The resulting DNA-rRNA-RNA complexes were immobilized by inserting an oligo(dT) coated dipstick into each solution for 5 min at 37°C. After removal, each dipstick was washed three times in 0.1 M phosphate buffer (pH 7) and then incubated for 10 min at room temperature with 10 µg/ml streptavidin (in phosphate buffer). The dipsticks were again washed three times with 0.1 M phosphate buffer (pH 7) and incubated for 10 min at room temperature with $10^{10}$ pfu biotinylated M13SOC512 bacteriophage (in phosphate buffer). The dipsticks were washed three times in 0.1 M phosphate buffer (pH 7) and digested with 1 µg of ribonuclease A for 1 hour at 37°C. The dipsticks were withdrawn and the supernatants for each dilution were assayed as described in Example 1.

| Listeria Cell Equivalents | Light Output (light counts/sec) | Signal:Noise |
|---|---|---|
| $4\times10^6$ | $2.23\times10^6$ | 284.0:1 |
| $4\times10^5$ | $8.69\times10^6$ | 111.0:1 |
| $4\times10^4$ | $7.52\times10^4$ | 9.6:1 |
| $4\times10^3$ | $7.46\times10^3$ | – |
| $4\times10^2$ | $3.15\times10^3$ | – |
| 40 | $5.27\times10^3$ | – |
| 4 | $4.43\times10^3$ | – |
| 0 | $7.85\times10^3$ | – |

The assay was sensitive to about $4\times10^4$ cell equivalents or about $3\times10^{-15}$ moles of rRNA.

## Example 3

This example illustrates the invention in a magnetic bead assay format for the detection of E. coli cells. The vector was a genetically modified $\phi$X174 bacteriophage. The phage, designated $\phi$X174 J-F-ins 6, had been previously modified as described by Müller et al., J. Mol. Biol., 141 :1-24 (1980). Biotin was bound to the surface of $\phi$X174 J-F-ins 6 following the protocol used to biotinylate the M13SOC512 bacteriophage of Example 1.

The detector probe comprised a synthetic DNA chain, biotin and streptavidin. The DNA contained a sequence of 35 nucleotides able to bind to the 16S rRNA from E. coli and to which 6 units of tetraethylene glycol were added to the 5' end. The nucleotide sequence was TGA AAG TAC TTT ACA ACC CGA AGG CCT TCT TCA TAC. Biotin was bound to the end tetraethylene glycol monomer.

The detector probe was prepared by reacting tetraethylene glycol (6.25 g) with 4,4'-dimethoxytrityl chloride (5.46 g) in anhydrous pyridine (70 ml) overnight at room temperature with stirring. The solvent was then removed in vacuo and the residue was twice coevaporated with toluene, dissolved in ethyl acetate, and twice extracted with saturated sodium hydrogen carbonate solution, dried ($MgSO_4$) and evaporated to dryness in vacuo. The residue was further purified by flash chromatography on silica gel using a gradient of ethyl acetate in hexane (1 :4, v/v to 1 :1, v/v) containing 2% triethylamine.

The reation product (2.42 g) was dissolved in dichloromethane (30 ml) containing N,N-diisopropyl ethylamine (1.109 g). Methoxy-N,N-diisopropyl-chlorophosphoramidite (1.107 g) was added dropwise to the stirred solution. The solution was stirred for 1 hour at room temperature. The mixture was diluted with ethyl acetate (prewashed with saturated sodium hydrogen carbonate solution) and twice extracted with saturated sodium hydrogen carbonate solution. The ethyl acetate layer was evaporated to dryness in vacuo. The product was further purified by flash chromatography on silica gel, packed with ethyl acetate :hexane :triethylamine (20 :80 :5, v/v/v) and eluted with the same solvent. The product was dissolved in acetonitrile to a 0.1 M solution for further coupling with an automated DNA synthesizer using protocols described by the manufacturer, Applied Biosystems, Foster City, CA.

The nucleotide chain was assembled using standard methods and commercially available adenosine, cytosine, guanosine and thymidine amidites.

The dimethoxytrityl protecting group was removed from the terminal 5'-hydroxyl group using 3% trichloroacetic acid in dichloromethane and six successive coupling, capping, oxidation and detritylation cycles were carried out with tetraethylene glycol phosphoramidite.

Phosphoramidite was prepared using methods similar to those described above and 3-(N-trifluoroacetylamino)propanol and methoxy-N,N-diisopropyl chlorophosphoramidite. Phosphoramidite was added and followed by capping and oxidation. The product oligonucleotide was removed from the controlled pore glass support and deprotected using standard methods. Biotin long chain (NHS-LC-Biotin, Pierce Chemical Co., Rockford, IL) was attached using standard methods.

The capture probe comprised a first polynucleotide with a sequence of nucleotides complementary to the 16S rRNA from E. coli. at residues 448 to 482 and a poly(dA) tail of about 150 dA residues. The capture probe also comprised a second polynucleotide with a poly(dT) sequence for binding to the poly(dA) tail of the first polynucleotide of the capture probe. Magnetic beads served as a solid phase as in Example 1. The second polynucleotide was bound to the magnetic beads as described in Example 1.

The sensitivity of the assay format for E. coli was determined as follows. Cell extracts from E. coli were serially diluted in 2.5 M GuSCN to about $10^8$, $10^7$ $10^6$, $10^5$, $10^4$ and $10^3$ cell equivalents of 16S rRNA per assay. The first polynucleotide of the rupture probe was added in 2.5 M GuSCN buffer to a cocentration of about $2 \times 10^{12}$ molecules per assay. The biotinylated synthetic DNA element of the detector probe was added in 2.5 M GuSCN buffer to a concentration of about $10^{12}$ molecules per assay. The total volume of each dilution was then about 100 μl. The dilutions were incubated for 60 min at 37°C to allow formation of a DNA-rRNA-polynucleotide complex.

Next, 50 μl samples of the dilutions were transferred to 12 mm x 75 mm polypropylene test tubes. One hundred microliters of the poly(dT) coated beads (0.1% solids and pretreated as in Example 1) were added to each tube. The tubes were incubated for 15 min at 37°C with shaking. The beads were then separated from the solutions and washed three times with wash buffer (0.5 M NaCl, 0.1 M Tris (pH 7.5), 0.5% BSA and 0.1% Tween 20). Each set of beads was combined with 200 μl of 1 μg/ml streptavidin in wash buffer and incubated for 10 min at room temperature.

The beads were again separated from the solutions and washed three times with wash buffer. $10^9$ pfu of biotinylated φX174 J-F-ins 6 in 200 μl wash buffer was added to 100 μl aliquots of beads. The solutions were incubated for 30 min at 37°C with shaking, separated from the beads and washed five times with 0.5 ml wash buffer.

Twenty units of RQI DNAse from Promega Biotech (Madison, WI) in 200 μl buffer (0.01 M NaCl, 0.04 M Tris, 0.006 M $MgCl_2$) was combined with the beads and incubated for 20 min at 37°C. The beads were removed and the supernatant was titered by plaquing on a lawn of bacteria. The results from each dilution are as follows:

| E. coli Cell Equivalents/Assay | Titer φX174/Assay | Signal:Noise |
|---|---|---|
| $5 \times 10^7$ | $4.0 \times 10^5$ | 181:1 |
| $5 \times 10^6$ | $1.2 \times 10^4$ | 5:1 |
| $5 \times 10^5$ | $5.2 \times 10^5$ | 236:1 |
| $5 \times 10^4$ | $3.5 \times 10^5$ | 159:1 |
| $5 \times 10^3$ | $2.4 \times 10^4$ | 11:1 |
| $5 \times 10^2$ | $8.0 \times 10^4$ | 36:1 |
| 0 | $3.2 \times 10^3$ | -- |

The assay was sensitive to about 500 E. coli cell equivalents or about $4 \times 10^{-17}$ moles of target.

Example 4

Example 4 illustrates the invention in a microtiter well assay format for the detection of Listeria innocua. The format was able to detect as few as 10 calls of the pathogen.

The vector was the genetically modified M13SOC512 bacteriophage used in Examples 1 and 2. An F(ab') monomer of anti-fluorescein antibody was bound to the surface of the phage instead of biotin as in Examples 1 and 2. The phage was first labeled wtih maleimide by incubating $10^{14}$ pfu of the phage with 3.4 mg sulfo-N(4-carboxycyclo-hexylmethyl) maleimide in 5 ml phosphate buffer. The product was purified by Sephadex G25 chromatography. F(ab)'$_2$ fragments of anti-fluorescein antibody were prepared by the standard pepsin digestion protocol. The dimeric fragments were reduced to monomers by incubating 2 hours with 0.015 M dithiothreitol under mildly acidic conditions (0.1 M sodium acetate, 0.1 M NaCl, pH 5.0). 0.1 mg F(ab)'monomer was incubated in 8 ml phosphate buffer at 25°C overnight with $10^{12}$ pfu of the modfied phage. The product was purified by cesium chloride density centrifugation (10 ml, 0.44 g CsCl/ml, 20 hours at 150,000 x g). The phage fraction was then dialyzed extensively against phosphate buffer.

The detector probe was prepared using a commercially available T7 RNA polymerase transcription kit (Promega, Madison, WI) according to manufacturer's instructions. Allylamine-UTP (Bethesda Research Laboratories) was substituted for UTP in the reaction mixture. The RNA transcript was fluorescein labeled by incubating 2.5 μg of transcript with 0.4 mg fluorescein isothiocyanate (Molecular Probes, Eugene, OR) in 200 μl borate buffer (0.1 M borate, pH 8.5) at 37°C for 2.5 hours.

The capture probe comprised a biotinylated RNA 713 nucleotides long and complementary to bases 674 to 1387 of the Listeria innocua ribosomal RNA. The probe was prepared using the Promega T7 RNA polymerase transcription kit. Biotin-11-UTP (Bethesda Research Laboratories) was substituted for UTP in the reaction mixture.

The walls of the microtiter wells served as a solid phase for the assay. CoBind microtiter wells from Micromembranes (Newark, NJ) were used. Streptavidin wn covalently bound to the microtiter wells as follows. Streptavidin in phosphate buffer was added to each well (0.1 ml, 5 mg/ml). The buffer was made up as 0.1 M phosphate/0.1 M NaCl, pH 7.5. The wells were incubated for 3 hours at room temperature and then washed with three 0.15 ml portions of phosphate buffer. The wells were then blocked with excess amino groups by adding 0.2 ml Tris-HCl/NaCl (0.1 M/0.1 M, pH 8.0) to each well and incubated overnight at 25°C. The wells were then washed with three 0.15 ml portions of phosphate buffer and stored damp at 4°C until use.

The sensitivity of the assay for Listeria innocua was determined as follows. Cell extracts of Listeria innocua were serially diluted to concentrations decreasing by factors of ten to 10 cells/ml. RNA-rRNA-oligonucleotide complexes were prepared for each dilution following the protocols of Example 1.

0.1 ml aliquots of the complexes were added to individual microtiter wells and incubated for 1 hour at 25°C. The wells were washed 3 times with wash buffer (0.1 M Tris, 0.5 M NaCl, 0.5% BSA, 0.1% Tween 20, pH 7.5). $10^{16}$ pfu of M13SOC512 bacteriophage with anti-fluorescein F(ab') antibody attached was added to each well in 100 μl wash buffer and incubated for 60 min at 25°C. The wells were then washed as above. Ribonuclease A (1 μg) was added and incubated for 60 min.

Aliquots of each test solution (50 μl) were added to E. coli host cells (50 μl). The cultures were grown for 2 hours at 37°C. Next, 20 μl of 0.04% M decanal in 2 YT broth was added. The microtiter wells were exposed to Polaroid film in a dark room for 5 min to record the emitted light. Emitted light was visible above background down to a concentration of 10 cell equivalents per assay or about $8 \times 10^{-19}$ moles rRNA.

## Example 5

This example also illustrates the invention in a microtiter well assay format for the detection of Listeria monocytogenes. The target was the 16S rRNA from the pathogen. The vector was the same M13SOC512 used in previous examples.

The detector probe comprised biotin, streptavidin and a synthetic polynucleotide chain complementary to bases 674 to 1387 of the 16S RNA of E. coli. The polynucleotide chain was prepared using conventional techniques. Biotin was bound to the chain during synthesis using conventional techniques. Streptavidin was first bound to the phage as follows. The phage was first reacted with sulfo succinimidyl-4-(N-maleimidomethyl)cyclohexane-1-carboxylase (Sulfo-SMCC ; Pierce Chemical Co., Rockford, IL) in 0.2 M MOPS buffer (3-[N-morpholino]propane sulfonic acid, pH 7.4) at room temperature for 1 hour. The molar ratio of SMCC :phage was 500 :1. The SMCC-phage conjugate was separated from unreacted sulfo-SMCC by Sephadex G-25 chromatography. N-succinimidyl 3-(2-pyridyldithio)propionate (SPDP ; Pierce Chemical Co.) was mixed with streptavidin (Calbiochem, LaJolla, CA) in 0.2 M MOPS at room temperature for 1 hour. The molar ratio of SPDP to streptavidin was 100 :1. The reaction mixture was purified by Sephadex G-25 chromatography. Dithiothreitol (Sigma Chemical) was added to the eluted protein to a final concentration of 50 mM. The reduced streptavidin-SPDP conjugate was purified by passage through a Sephadex G-25 column. The SMCC-phage was reacted with streptavidin-SPDP (molar ratio of 1 :50) for 16 hours at 4°C. The streptavidinylated phage was then purified on a cesium chloride density gradient (0.44 g/ml ; 20 hours, 150,000 x g, 4°C), dialyzed against 0.2 M MOPS buffer, pH 7.5 and stored at 4°C until use.

The capture probe comprised the same dA-tailed 35 base nucleotide used in Example 1 and a 4000 base dT homopolymer (dT 4000 ; Supertechs, Inc., Bethesda, MD). The dT 4000 was attached to Immulon 2 microtiter wells from Dynatech, Inc., Chantilly, VA, by adding 250 μl of dT 4000 (100 μg/ml in 1.5 M NaCl, 0.5 M MgCl$_2$, 0.25 M Tris-HCl, pH 7.5) and incubating at 37°C overnight. After removal of the remaining dT 4000 solution, the wells were dried at 37°C for 15 to 30 min, irradiated with UV light for 4 min and washed 3 times with wash buffer (1 M NaCl, 0.1 M Tris-HCl, pH 9.3, 2 mM MgCl$_2$ and 0.1 % Tween 20).

The wells were treated for 1 hour at 37°C with 1.0 M NaCl, 0.2 M Tris-HCl, pH 7.5, 0.04 M EDTA, 2.5% bovine serum albumin (fraction V BSA), 0.5% sarkosyl (Sigma Chemical) and 10 µg/ml yeast tRNA (Bethesda Research Labs). The wells were then washed 3 times with wash buffer, dried and stored in a desiccator in the dark until use.

The sensitivity of the assay was determined as follows. To separate 1.5 ml polypropylene tubes were added extracts from 1000, 10, 1 and 0.1 cell equivalents of Listeria monocytogenes, 100 ng of the dA-tailed oligonucleotide of the capture probe and 50 ng of the biotinylated polynucleotide chain of the detector probe. The contents of the tubes were brought to 200 µl by adding 0.5 M NaCl, 0.1 M Tris-HCl (pH 7.5) solution. The tubes were heated at 65°C for 30 min to allow hybridization to occur.

Following hybridization, $10^9$ pfu of the streptavidinylated bacteriophage was added to each tube. The tubes were then incubated for 60 min at 37°C with shaking. The contents of each tube were then transferred to separate dT 4000 coated microtiter wells and then incubated for 60 min at 37°C with mild shaking. The contents of the wells were then washed with wash buffer (0.5 M NaCl, 0.1 M Tris-HCl, pH 7.5). The contents were then incubated with 200 µl of 0.01 M Tris-HCl, 0.04 M NaCl, 0.006 M $MgCl_2$, pH 7.5 containing 20 units of RP1 DNAse (Promega) to release the phage from the microtiter wells. The incubation was for 30 min at 37°C.

Aliquots (100 µl) of each mixture were added to 200 µl portions of a mid-log E. coli JM101 culture grown in TYE broth containing 0.5 mM isopropyl-β-D-thiogalactoside (IPTG). The mixtures were incubated for 10 min at room temperature. The mixtures were then further diluted with 2 ml of the same TYE broth and incubated for 2.5 hours at 37°C with vigorous shaking. Decanal (10 µl, 0.2%) was added to 100 µl samples of each mixture and the resulting luminescence was measured. The result for each dilution was :

| *Listeria* Cell Equivalents | Light Output *(light counts/sec)* | Signal:Noise |
|---|---|---|
| 1000.0 | 9.66x10⁴ | 6.0:1 |
| 10.0 | 6.62x10⁴ | 4.1:1 |
| 1.0 | 4.02x10⁴ | 2.5:1 |
| 0.1 | 3.20x10⁴ | 2.0:1 |
| 0.0 | 1.61x10⁴ | -- |

The assay was sensitive to less than about 0.1 cell equivalents or about $8x10^{-21}$ moles of target.

The Listeria innocua and Listeria monocytogenes strains referenced in the specification were deposited with American Type Culture Collection (ATCC) in Rockville ; Maryland, U.S.A. , and have been assignated ATCC Accession Nos. ATCC 33090 and ATCC 15313 ; respectively.

## Claims

1. A self-amplifying signal generating moiety for use in an assay to determine the presence or concentration of a target, said self-amplifying signal generating moiety comprising :
   (a) a vector which can be received by a host and reproduce thereby, and said vector is an active bacteriophage, virus, plasmid or fragment thereof ; and
   (b) a detector probe able to bind said vector and the target of said assay.

2. The self-amplifying signal generating moiety of claim 1 wherein said detector probe consists of one or more components selected from the group consisting of antibodies, streptavidin, avidin, biotin, oligonucleotides, Fab′ fragments, Fab′ fragments and F(ab′)₂ fragments.

3. The self-amplifying signal generating moiety of claim 1 wherein said vector encodes for a polypeptide.

4. The self-amplifying signal generating moiety of claim 3 wherein said vector is an active M13 or φX174 bacteriophage.

12

5. The self-amplifying signal generating moiety of claim 3 wherein said detector probe consists of one or more components selected from the group consisting of antibodies, streptavidin, avidin, biotin, oligonucleotides, Fab fragments, Fab' fragments and $F(ab')_2$ fragments.

6. The self-amplifying signal generating moiety of claim 3 wherein said polypeptide is a luciferase enzyme.

7. A method for determining the presence or concentration of a target molecule comprising the steps of :
    (a) contacting a vector and a detector probe with a sample to be tested for said target molecule wherein said vector is a member of the group consisting of an active bacteriophage, virus, plasmid or fragment thereof, and which can be received by a host and reproduce thereby, and said detector probe is able to bind to said vector and said target molecule ;
    (b) allowing formation of a vector-detector probe-target complex ;
    (c) separating said vector-detector probe-target complex from uncomplexed vector, detector probe and target ;
    (d) introducing the vector from said separated vector-detector probe-target complex to a host whereby said vector is able to replicate ;
    (e) allowing said replicating vector to produce a signal ; and
    (f) detecting said signal.

8. The method for determining the presence or concentration of a target molecule of claim 7 wherein said detector probe consists of one or more components selected from the group consisting of antibodies, streptavidin, avidin, biotin, oligonucleotides, Fab fragments, Fab' fragments and $F(ab')_2$ fragments.

9. The method for determining the presence or concentration of a target molecule of claim 7 wherein said vector-detector probe-target complex is bound to a solid phase support before being separated from said uncomplexed vector, detector probe and target.

10. The method for determining the presence or concentration of a target molecule of claim 9 wherein a capture probe is used to bind said vector-detector probe-target complex to said solid phase support.

11. The method for determining the presence or concentration of a target molecule of claim 10 wherein said capture probe consists of one or more components selected from the group consisting of antibodies, streptavidin, avidin, biotin, oligonucleotides, Fab fragments, Fab' fragments and $F(ab')_2$ fragments, and said solid phase support is a microtiter well, a cup or a dipstick.

12. The method for determining the presence or concentration of a target molecule of claim 7 wherein said vector encodes for a polypeptide.

13. The method for determining the presence or concentration of a target molecule of claim 12 wherein said detector probe consists of one or more components selected from the group consisting of antibodies, streptavidin, avidin, biotin, oligonucleotides, Fab fragments, Fab' fragments and $F(ab')_2$ fragments.

14. The method for determining the presence or concentration of a target molecule of claim 12 wherein said vector-detector probe-target complex is bound to a solid phase support before being separated from said uncomplexed vector, detector probe and target.

15. The method for determining the presence or concentration of a target molecule of claim 14 wherein a capture probe is used to bind said vector-detector probe-target complex to said solid phase support.

16. The method for determining the presence or concentration of a target molecule of claim 15 wherein said capture probe consists of one or more components selected from the group consisting of antibodies, streptavidin, avidin, biotin, oligonucleotides, Fab fragments, Fab' fragments, $F(ab')_2$ fragments, and said solid phase support is a microtiter well, a cup or a dipstick.

17. The method for determining the presence or concentration of a target molecule of claim 16 wherein said polypeptide is a luciferase enzyme.

18. An assay kit for determining the presence or concentration of a target in a sample comprising :

(a) a vector which is a member of the group consisting of an active bacteriophage, virus, plasmid or fragment thereof, which is able to produce a signal, and which can be received by a host and reproduce thereby ;

(b) a detector probe able to bind said vector and the target of said assay ;

(c) means for accessing said sample with said vector and said detector probe ;

(d) means for separating vector and detector probe which are bound to said target from vector and detector probe which are not bound to said target ; and

(e) means for introducing said separated bound vector to a host able to receive said vector and permit reproduction thereof.

19. The assay kit of claim 18 wherein said kit further comprises a host able to receive said vector and permit reproduction thereof.

20. The assay kit of claim 18 wherein said vector encodes for a polypeptide.